# EUROPEAN PATENT APPLICATION

(11) **EP 1 087 230 A1**
(43) Date of publication of application: **28.03.2001**
(21) Application number: 99118847.5
(22) Date of filing: 24.09.1999
(51) Int. Cl.: G01N 33/573, G01N 33/68, C12N 9/12, C07K 14/47, C12Q 1/48

(54) **Method for identifying compounds useful in the therapy of bone disorders**

(71) Applicant: Boehringer Ingelheim International GmbH, 55218 Ingelheim am Rhein (DE)
(72) Inventor: David, Jean-Pierre, 1020 Wien (AT); Sabapathy, Kanaga, 1020 Wien (AT); Behrens, Axel, 8032 Zürich (CH); Wagner, Erwin F., Prof. Dr., 1030 Vienna (AT)
(74) Representative: Laudien, Dieter, Dr.

(57) **Abstract**

A method for identifying a compound useful for the treatment of bone disorders caused by osteoclast differentiation or activation that leads to increased bone resorption, wherein the compund is tested for its ability to inhibit JNK1 synthesis or JNK1 activation and/or c-Jun N-terminal phosphorylation.

## Description

The present invention relates to the therapy of bone disorders that are caused by increased bone resorption, in particular osteoporosis.

Bone is an organ subject to permanent remodelling during development and adult life of a vertebrate. This remodelling is the result of combined activities of two main cell lineages: osteoblasts, the bone forming cells and osteoclasts, the bone resorbing cells. These two cell types tightly interact with each other providing signals which control their differentiation and activation.

Osteoporosis is a systemic disease characterized by low bone mass and microarchitectural deterioration in the entire skeleton with a consequent increase in bone fragility and susceptibility to fracture, especially of bones subjected to major mechanical forces. Osteoporosis develops when the rate of bone resorption exceeds the rate of bone formation resulting in a progressive loss of bone mass. A large number of risk factors for osteoporosis have been identified including aging and loss of gonadal function. In addition, osteoporosis is associated with various endocrine, haematologic, gastrointestinal and rheumatologic diseases, and can be the consequence of therapy with glucocorticoids, heparin, and antiepileptic drugs. The major clinical manifestation of osteoporosis are vertebral body fractures, leading to pain in the back and deformity of the spine. The diagnosis of osteoporosis is based on reduced bone mass, usually assessed by measuring bone mineral density. Most of the drugs used to treat osteoporosis act by decreasing bone resorption, including estrogens, bisphosphonates, and calcitonin.

It was an object of the invention to provide a method for identifying compounds that interfere with the differentiation or activation of osteoclasts and are therefore useful in the therapy of bone disorders caused by increased bone resorption, in particular for the treatment of osteoporosis.

To solve the problem underlying the invention, the cellular and molecular mechanisms underlying osteoclast differentiation were studied.

Osteoclasts are derived from hematopoetic precursors within the monocyte-macrophage lineage and their differentiation is under the control of two cytokines: macrophage stimulating factor (mCSF) and tumor necrosis factor-related activation induced cytokine (TRANCE), both of which are synthesised by osteoblasts and act on osteoclast progenitors (Takahashi et al., 1999). During this process, the precursor cells progressively express a number of phenotypic markers of the mature osteoclast including tartrate-resistant acid phosphatase, the vitronectin receptor (integrin αᵥβ₃), the calcitonin receptor, and carbonic anhydrase II (CA II). In most cases, the mechanisms responsible for the enhanced expression of genes during osteoclast differentiation have not been fully elucidated. The targeted disruption of the c-*fos* or the double deletion of two members of NFκB family result in severe forms of osteopetrosis (Wang et al., 1992) (Franzoso et al., 1997) which is due to a block in osteoclast differentiation and, in the case of c-*fos*, results in an accumulation of early progenitors and macrophages in the marrow (Grigoriadis et al., 1994). The fos-related-antigen-1 protein, Fra-1, another member of the Fos family of transcription factors (Cohen and Curran, 1988), and a putative target of c-Fos (Braselmann et al., 1992), displays a strong osteoclastogenic activity when overexpressed *in vivo* or *in vitro* in osteoclast progenitors (Owen et al., 1999). These observations indicate that Fos and NFκB are required for commitment to and/or progression in the osteoclast lineage. Fos belongs to the group of transcription factors called AP-1 which are formed by heterodimerization of a member of the Fos family (c-Fos, FosB, Fra-1 or Fra-2) and a member of the Jun family (c-Jun, JunB or JunD). AP1 transcriptionaly modulates the expression of genes through the binding to a consensus sequence called tetradecanoyl-phorbol acetate (TPA)-responsive element (TRE) or AP1 binding site (Angel and Karin, 1991). By analogy with the known action of mCSF in macrophages, where its binding to the receptor tyrosine kinase CSF-1R encoded by the c-*fms* protoconcogene can both activate the pool of available c-Fos (immediate early response) and upregulate c-Fos expression (delayed early response) (Sherr, 1991), c-Fos could be proposed as the downstream effector mediating mCSF action in osteoclast differentiation. In contrast, until now, none of the Jun members or the signalling pathway leading to Jun activation have been clearly implicated in osteoclast differentiation or activation. Jun transcriptional activity can be modulated by various extracellular signals including growth factors, ultraviolet, stress, osmotic shock and imflamatory cytokines leading to the activation of the Jun-N-terminal kinases cascade (Davis, 1994). JNKs protein kinases are able to modulate the activation of two of the members of the Jun family, c-Jun and JunD (Gupta et al., 1996) through the phosphorylation of conserved motif, serine 63 and 73 for c-Jun (Gupta et al., 1996).

Recently, the osteoclast differentiating factor (ODF) has been identified to be TRANCE (Wong, et al., 1997; Darnay et al., 1999), also called osteoprotegerin ligand (OPGL) or receptor activator of NFκB ligand (RANKL) (Lacey et al., 1998; Yasuda et al., 1998). TRANCE mediated-osteoclastogenesis can be blocked by the binding to the extracellular decoy receptor, osteoprotegerin (OPG) (Simonet et al., 1997). TRANCE positively acts through the binding to the membrane-bound TNF-related receptor, RANK, leading to a tumor necrosis associated factors (TRAFs) dependent activation of NFκB or of the Jun N-terminal kinases (JNKs) (Anderson et al., 1997; Darnay et al., 1999; Galibert et al., 1998; Wong et al., 1997). Targeted deletion of TRAF6 impairs the activation process of the osteoclasts but does not affect their differentiation, resembling to the phenotype previously described for the c-*src* null allele (Soriano et al., 1991) (Lomaga et al., 1999), and to a lesser extent to some of the natural mutations of the transcription factor microphtalmia.

In the experiments of the present invention, the role of the Jun signalling cascade, more specifically the activation of JNKs, in osteoclast differentiation was examined.

In the experiments of the present invention, mice lacking JNK1 or JNK2, and mice homozygous for a mutated c-*jun* Allele (*jun* AA) that cannot be phosphorylated at serine 63, 73 by the JNKs were generated. These mice are viable and do not develop any obvious osteopetrotic phenotype although two of them, *jnk1* -/- and *jun* AA, have a smaller body size. To characterize the importance of JNK activation in osteoclast differentiation, hematopoetic precursors were isolated from both spleen and bone marrow of the mutant mice and osteoclasts were generated in coculture on wild type primary osteoblasts. Osteoclasts derived from *jnk2* -/- have identical differentiation properties and resorptive activity as wild-type osteoclasts. In contrast, homozygous deletion of *jnk1* results in a decrease in bone resorption activity due to a 50% decrease in osteoclast numbers. Similarly, osteoclast differentiation is also affected when hematopoetic precursors were isolated from the *jun* AA mice, suggesting that c-Jun phosphorylation by JNK1 but not JNK2 is necessary for efficient osteoclastogenesis. Immunoprecipitationkinase assays demonstrate that JNK1 but not JNK2 is activated upon TRANCE stimulation of macrophage colony stimulating factor dependent macrophage-osteoclast precursors isolated from wild-type bone marrow and spleen hematopoetic cells, resulting in an induction of AP1 DNA binding activity.

It could be shown that TRANCE specifically activates JNK1 but not JNK2 in osteoclast progenitors. JNK1 but not JNK2 is required for a fully efficient differentiation of osteoclasts through the phosphorylation of c-Jun. Finally it was demonstrated that TRANCE induce AP1 but not NFκB binding activity in osteoclast progenitors. It may therefore be concluded that JNK1 activation is involved in mechanisms leading to a pathological increase of osteoclast differentiation such as the postmenopausal osteoporosis, underlining the importance of developing specific JNK-1 inhibitors or cell specific targeted JNKs inhibitors.

To summarize, the results obtained in the experiments demonstrate that JNK1 activity is induced in the osteoclast lineage upon TRANCE stimulation. JNK1 but not JNK2 can modulate osteoclastogenesis efficiency and it can therefore be concluded that JNK1 modulates osteoclast differentiation in response to TRANCE stimulation through phosphorylation of cJun and the induction of AP1 activity.

Based on these findings, the problem underlying the present invention was solved by providing methods for identifying compounds that are useful for the treatment of bone disorders caused by osteoclast differentiation or activation that leads to increased bone resorption, characterized in that the compounds are tested for their ability to inhibit JNK-1 synthesis or JNK1 activation and/or c-Jun N-terminal phosphorylation.

In the following, in the context of inhibition, the term "JNK1 activity" will be be used as a synonym for "JNK-1 synthesis or JNK1 activation".

Methods useful in the invention may be selected from any methods known in the art for screening compounds, e.g. peptides or organic compounds, that interfere with the activity of JNKs. Methods for identifying inhibitors of JNK1 activity that are useful in the present invention are described in WO 99/18193, the disclosure of which is fully incorporated herein by reference. These methods, which refer to JNK3 inhibitors, can be easily adapted for the application on JNK1.

The methods of the invention are useful to identify JNK1 inhibitors which may act by binding to JNK1, binding to intracellular proteins that bind to JNK1, interfering with the interaction between JNK1 and its substrates, modulating the activity of a JNK1 gene, or modulating the expression a JNK1 gene on the transcriptional or postranscriptional level. In addition, the methods of the invention are useful to identify compounds that interfere with the phosphoryation of a JNK1 substrate, e.g. c-Jun

In a first embodiment of the invention, methods are provided that are based on cell free assays.

In a first aspect, the assay is a binding assay.

The principle of such an assay is to detect the inhibition of the physical interaction of JNK1 with one of its substrates to which it binds, e.g. c-Jun (other known substrates are ATF2 and NF-AT4). Since the JNK binding domains for c-Jun have a high degree of homology and both the JNK binding domain on c-Jun, the δ-domain, and the c-Jun binding domain on JNK1 have been identified and characterized (Kallunki, et al., 1994; Gupta, et al., 1996), the biochemical tools for the assay are easily available.

The assay is conducted by incubating the JNK1 binding domain of the substrate, e.g. wildtype c-Jun, and the substrate binding domain on JNK1, one of them being fused to a detectable reporter molecule, e.g. luciferase, and the other one being immobilized on a suitable carrier, e.g. micro titer plates, with the test substance and by determining the effect of the compound on the interaction of the two proteins by measuring the reporter activity. One or both binding partners may carry a tag resulting from the antecedent purification step, e.g. a GST, His or Myc tag, provided binding of the partners is not impaired by the tag. In this case, the protein may be specifically immobilized on the carrier via that tag. If a compound is an inhibitor of JNK1/c-Jun interaction, the reporter signal which results from binding of the two domains to one another, is decreased or abolished. Alternatively, larger proteins containing the binding domains may be used, e.g. the full length proteins, or one of the interaction partners as a full-length protein and the other as the binding domain only, preferably the c-Jun N-terminal peptide and the full length JNK.

In a first embodiment of such an assay, JNK1, or alternatively the Jun binding domain of JNK1, is recombinantly produced by conventional methods, by expressing the cDNA encoding JNK1 (Derijard et al., 1994) or the binding domain (Gupta, et al., 1996) in a suitable host. Next, the c-Jun N-terminus, encompassing approximately the N-terminal 100-200 amino acids, is fused to a reporter molecule, e.g. lacZ or the luciferase, preferably GFP. This fusion protein can be obtained by recombinant DNA technology as follows: the DNA sequence encoding the c-Jun N-terminus (Hattori, et al., 1988) is ligated to the reporter gene sequence and inserted into a suitable expression vector, e.g. in a bacterial plasmid, and expressed in the host. Alternatively to using the c-Jun N-terminus, other known or yet to be identified substrates that bind to JNK1 can be employed, using either the entire protein, or preferably a fragment that comprises or consists of the JNK1 binding domain.

Alternatively, the reporter protein can be fused in an analogous manner to the JNK1 protein substrate, e.g. c-Jun, binding domain, respectively.

Alternatively, one of the interaction partners may be labeled by any other detectable marker, preferably a marker producing a signal that can be easily measured in a high throughput screen, in which the unlabeled interaction molecule is immobilized, e.g. on a microtiter plate surface or on beads. Examples for suitable labels are radioactive labels or commercially available fluorescent markers suitable for labeling proteins or peptides. Furthermore, the assay may be carried out as a proximity assay using commercially availabe ingredients. Alternatively, one of the interaction partners may be conjugated to an enzyme, e.g. alkaline phosphatase or luciferase, which allows for monitoring the interaction by determining the enzymatic activity.

This assay type may be performed in the high-throughput format using known methods; e.g. those described in WO 99/18193. It is useful for finding drugs the therapeutic effect of which is due to their blocking the interaction of JNK1 with one its substrates, e.g. the wild type c-Jun , e.g. by binding to JNK-1. Furthermore, this assay will identify compounds that block the interaction of the c-Jun N-terminal region with a kinase and thus interfere with c-Jun phosphorylation.

Molecules that block the interaction between JNK1 and its substrates (or the c-Jun N-terminal region with a kinase) can also be identified using the yeast two-hybrid method as described in WO 99/18193.

Examples for compounds whose effect is based on blocking JNK1 activity, are peptides or peptidomimetics that compete with the natural substrate for binding to JNK1, e.g. a fragment (Adler et al., 1994) or a mutated version of the natural substrate (e.g. c-Jun having serine 63 and serine 73 mutated, see Example 1). An example for a compound the effect of which is based on blocking c-Jun N-terminal phosphorylation is a peptide or peptidomimetic of a JNK which binds but does not phosphorylate the c-Jun N-terminus, e.g. dominant-negative JNK or a fragment thereof. (This JNK may either be JNK1 or another kinase phosphorylating serine 63 and serine 73 of c-Jun, e.g. JNK2, JNK3.)

To verify the specificity of the compounds which have shown an inhibiting effect on substrate/JNK1 interaction, additional JNKs, e.g. JNK2 or JNK3, are tested for their interaction with the substrate in the presence or the absence of the compound. Specific inhibitors abolish JNK substrate binding to JNK1, but not the binding of other JNKs (Gupta, et al., 1996).

In a second aspect, the method of the invention is based on a cell-free kinase assay that directly determines the modulating effect of the test compound on the phosphorylation of c-Jun (or another JNK1 substrate) by JNK1.

The principle of such an assay is to inhibit the phosphorylation of the substrate, e.g. the N-terminal phosphorylation of c-Jun, by JNK1. The relevant phosphorylation sites in c-Jun have been identified as serine 63 and serine 73 (Derijard, *et al*., 1994; Kyriakis *et al*., 1994). The biochemical tools for the assay are readily available.

The assay is conducted by incubating recombinant JNK1 (Derijard et al., 1994) and the recombinant JNK1 substrate, e.g. c-Jun, in the presence of adenosine triphosphate (ATP), c-Jun being immobilized on a suitable carrier, e.g. microtiter plates, with the test compound and by determining the effect of the compound on the kinase reaction by measuring the incorporation of phosphate into the substrate. One or both binding partners may carry one or more tags resulting from the antecedent purification steps, e.g. a GST, His or Myc tag, provided the phosphorylation of the substrate is not impaired by the tag.

In this case, the protein may be specifically immobilized on the carrier via that tag. If a compound is an inhibitor of the phosphorylation by JNK1, the reporter signal which results from the phosphorylation of c-Jun is decreased or abolished.

Alternatively, a region of the substrate that comprises the JNK phosphorylation sites may be used as a substrate.

In a first variation of such an assay, JNK1 is recombinantly produced by conventional methods, by expressing the cDNA encoding the kinase in a suitable host. A cDNA encoding the full-length substrate or alternatively the region containing the phosphorylation sites, in the case of c-Jun encompassing approximately the N-terminal 100-200 amino acids, is inserted into a suitable expression vector , e.g. in a bacterial plasmid, and expressed in the host.

The substrate protein is immobilized to a carrier, e.g. on a microtiter plate surface or on beads. This assay is particularly suitable for the high-throughput format.

By way of example, the kinase reaction is carried out in the presence of synthetic ATP. The phosphorylation of the relevant serines 63 and 73 of the immobilized substrate c-Jun is detected by a c-Jun phosphoserine 63-specific or a c-Jun phosphoserine 73-specific antibody that is directly conjugated with a reporter molecule, e.g. alkaline phosphatase or horse radish peroxidase. Alternatively, the binding of the c-Jun phosphoserine 63-specific or a c-Jun phosphoserine 73-specific antibody is detected by a species-specific secondary antibody that is conjugated with a reporter. Alternatively, this type of assay may be conducted for other JNK1 substrates, e.g. for ATF2, for which antibodies are commercially available.

Alternatively, the kinase reaction can be carried out in the presence of trace amounts of γ-phosphate (³²P or ³³P) radioactive labelled ATP. The progression of the kinase reaction is followed by the incorporation of radioactive phosphorus into the substrate, e.g. in a scintillation proximity assay format. This assay is particularly suitable for the high-throughput format.

Other methods for carrying out kinase assays are available, e.g. in-gel protein kinase assays using the substrate GST-cJun, as described in WO 99/18193.

In analogy to the binding assay described above, the kinase assay is useful for identifying both compounds that block JNK1 activity and compounds that interfere with c-Jun N-terminal phosphoryation. Like JNK1 inhibitors, compounds that decrease the *in vitro* phosphorylation of the substrate, e.g. the N-terminal part of c-Jun, are drug candidates for the treatment of bone disorders associated with a level of osteoclast differentiation that causes detrimental bone resorption.

To verify the specificity of the substances which have shown an inhibiting effect on phosphorylation by JNK1, additional JNKs, e.g. recombinant JNK2 or JNK3, are tested under the same conditions in the presence or the absence of the test compound. Specific inhibitors inhibit phosphorylation by JNK1, but not of other JNKs (Gupta, et al.,1996).

In an alternative embodiment, the screening methods of the invention are cellular assays.

On a type of cellular assays is useful for identifying compounds that lead to a down regulation of JNK-1 at the transcriptional level or post-transcriptional level. Such a screening method is based on mammalian cells, preferably human cells, in which the expression of JNK1 upon incubation with the test compound can be monitored. This screening method of the invention is based on a type of assay known in the art for identifying compounds that transcriptionally modulate the expression of a gene of interest. It may designed and carried out according to known methods, e.g. as described in WO 91/01379.

In this assay, which is well suited for use in a high-throughput format in order to test a great number of chemical compounds, inhibitors of JNK1 expression that act on the JNK1 regulatory region can be identified using a reporter construct in which sequences including the JNK1 regulatory region are fused to a reporter gene, for example firefly luciferase. Cultured cells, e.g. fibroblasts, transfected with this reporter construct are screened for the reporter activity, e.g. luciferase activity in this high throughput assay.

The effect of the compound can be confirmed by direct measurement of the endogenous JNK1 protein (by Western blotting) and JNK1 mRNA (by Northern blotting) using methods known in the art (for example, see Ausubel et al., 1994).

In an alternative embodiment of the invention, a screening method is provided which is based on mammalian cells, preferably human cells, in which the modulation of c-Jun N-terminal phosphorylation or JNK1-dependent activation of a substrate can be monitored. Also in these assays, compounds will be identified which interfere with JNK1 activity or with c-Jun N-terminal phosphorylation.

One way of monitoring c-Jun N-terminal phosphorylation or JNK1-dependent substrate activation is determining the expression of a reporter gene under the control of a regulatory element of a c-Jun target gene, e.g. fasL or collagenase I, or a target gene of another JNK1 substrate, which is directly regulated by this mechanism.

By way of example, test cells are used which carry a regulatory element of a c-Jun target gene fused to a reporter gene whose expression can be easily measured. In the case of fasL, the promotor region including the AP1 binding site, as described *inter alia* by (Faris, *et al*., 1998; Kasibhatla, 1998; Herdegen, 1998; Nagata, 1997), is known and easily available for the person skilled in the art. Alternatively, regulatory sequences of other c-Jun target genes may be employed. Such target genes can be identified by subjecting cells, e.g. osteoclasts or osteoclast progenitors, derived from junAA mice or *jnk1* -/- mice and control mice, to methods known in the art for determining differential gene and protein expression, e.g. differential display RT-PCR analysis (Liang and Pardee,1992;Bauer, et al., 1993), DNA microarray technology (DeRisi, 1997; Schena, 1995; Wodicka, et al., 1997, Ramsay, 1998), subtractive hybridization (Diatchenko, et al. 1996; Hubank and Schatz, 1994), or proteome analysis (Pennington, 1997; Pasquali, et al., 1997). Upon identification of the genes, the regulatory sequences and the AP1 binding sites or binding sites for JNK1 substrates may be obtained by conventional promotor analysis, which involves mutagenesis of these consensus binding sequences and measuring reporter gene activation depending c-Jun phosphorylation or on JNK1 substrate phoshorylation.

An example for conducting a screening assay based on this principle is the following:

In a first step, suitable test cells are selected, i.e. cells which can be easily grown in tissue culture and in which the activation of a reporter gene is dependent on the JNK1 signaling pathway and c-Jun N-terminal phosphorylation, can be measured. In a preferred embodiment, fibroblasts, which have been extensively used to study the JNK signalling pathway, are used as test cells.

The test cells are wild type cells stably transfected, by conventional techniques (Sambrook, et al., 1989), with a recombinant DNA molecule containing the regulatory region of a target gene for c-Jun N-terminal phosphorylation, e.g. fasL, fused to a reporter gene according to known methods.

Any reporter gene may be used whose expression product is detectable by measuring a signal proportional to its concentration and which can be detected and quantified. Preferably, a reporter gene is employed whose expression can be measured with high sensitivity in an automated assay.

Examples for preferred reporter genes are the luciferase gene (De Wet, et al., 1987), the Green Fluorescent Protein GFP (Inouye, and Tsuji, 1994), and the lacZ gene.

The cells are grown in a suitable medium in microtiter assay plates and induced to activate the JNK pathway, e.g. by UV or TNF-α, in the presence or absence of the compounds to be tested. Activation of the JNK pathway results in reporter gene activity which is expected to be downregulated by compounds which exhibit the desired effect, i.e. the ability to inhibit c-Jun N-terminal phosphorylation or to inhibit JNK1 acitivity. In a preferred embodiment, cells, e.g. fibroblasts, are used that have been engineered by known methods to express a receptor whose ligand is known to induce osteoclast differentiation, e.g. the receptor for TRANCE, i.e. RANK (Anderson et al., 1997). The engineered cells are incubated in the presence of the receptor ligand.

In order to determine the mechanism by which the test compound induces modulation of the reporter gene expression, i.e. to determine whether the effect is due to JNK1 inhibition or to c-Jun N-terminal phosphorylation, a variation of the screen can be employed in which fibroblasts, optionally engineered to respond toTRANCE, as described above, are used that are derived from *jnk1* -/- mice on the one hand and from junAA mice on the other hand. In these cells, a reporter gene is used that is either under control of an AP1 binding site (for the c-Jun phosphorylation) or under control of binding sites of another JNK substrate (for JNK1 inhibitors). By this assay, it can be determined whether a substance acts on the JNK pathway independently of c-Jun phosphorylation or acts on c-Jun phosphorylation independently of JNK1, thus determing the specifity of the substance for c-Jun phosphorylation.

In a preferred embodiment, an assay is employed which is capable of specifically monitoring the effect of a compound on osteoclast differentiation and activation.

The cellular screening methods of this type can be based on the so-called "co-culture" method in which osteoclast progenitors (hematopoietic cells from bone marrow or spleen cells) are cocultured and induced to differentiate on osteoblastic (mesenchymal) supportive cells (Akatsu et al., 1992). The supportive cells can be either wild type osteoblasts isolated from calvaria of new born animals or supportive cells, e.g. cells from the osteoblastic cell line P1-15 or the stromal cell line ST2 . Alternatively, a cell line (e.g. fibroblasts) can be used that has been modified to produce constitutively or in an inducible manner the cytokines necessary for the supportive function (mCSF and TRANCE). This can be achieved by transfection or infection of cells with known methods, e.g. with recombinant retrovirus.

In this type of assay, compounds that block preferentially the activation of JNK-1 or the phosphorylation of c-Jun N-terminal portion leading to a decrease of osteoclast differentiation or activation are considered as positive.

The differentiation of the osteoclasts is monitored by detecting the expression of osteoclast specific markers according to known methods, e.g. TRAP staining and/or the resorption activity of the cell (Akatsu et al., 1992).

This method monitors the activity of osteoclasts by assays on ivory or cortical bone slices, or on a commercially available synthetic substrate, e.g. hydroxyapatite coated discs, that allow automatisation of the read-out.

An alternative screening method directly uses mCSF dependent in culture hematopoietic osteoclast precursors (as described in the Examples) which are stimulated by TRANCE. Compounds that decrease or block JNK-1 activation or c-Jun phosphorylation and thus cause a decrease in osteoclast differentiation or function, monitored as described above, are positive drug candidates.

To carry out a pre-screen for the test compounds, cell lines responsive to TRANCE (Wong et al., 1997); e.g. monocytic cell lines that have been selected for the expression of RANK or cells engineered to express RANK (Nakagawa et al., 1998) may be used that differentiate to osteoclast like cells in response to TRANCE. The advantage of this method is that it can be carried out in a large scale.

From the results of the experiments of the present invention, it can be concluded that JNK1 activation is involved in mechanisms leading to a pathological increased differentiation of osteoclasts such as occuring in the postmenopausal osteoporosis. It is important to note that the basal level of differentiation of osteoclasts does not seem to be affected in the JNK-1 knock out mouse as in the Jun AA knock-in mice. The only effect of these genetic modifications that is seen, is a decrease of the efficiency of differentiation leading to the conclusion that JNK-1 and c-Jun phosphorylation are involved in pathogenic situation in which high level of differentiation of osteoclast occur. These include, osteoporosis post-menopausal, and other diseases like Padget disease, osteolytic metastasis, and osteoporosis associated to long term treatment with anti-imflamatory agent of the family of the corticosteroides. This observation is of importance because it implies that: blocking the phophorylation of c-Jun and/or the activation, synthesis of JNK-1 or its interaction with c-Jun will lead to a block osteoclast differentiation at a level that is detrimental without affecting the normal level of differentiation and so eliminating a lot of potential side effects.

The compounds that can be screened by the methods of the invention and can thus useful as drugs for the treatment of bone disorders associated with bone resorption include, but are not limited to, peptides and other organic compounds (e.g., peptidomimetics) that bind to a JNK1 protein or inhibit its activity in any way or which inhibit the N-terminal phosphorylation of c-Jun.

A further example for useful therapeutic compounds are JNK1 antisense nucleic acid molecules or ribozymes. The requirements on antisense RNA or DNA molecules complementary to JNK1 mRNAs such that they inhibit JNK1 translation or ribozymes that catalytically cleave JNK1 mRNAs are well known in the art, they can be synthesized and administered by standard methods, as described in WO 99/18193.

If a test compound has been identified to have an effect in a primary screen which is based on a non-cellular binding assay or in a kinase assay or in a cellular system which does not monitor osteoclast differentiation, a secondary assay is used to confirm whether the compound has an effect on osteoclast diffentiation or activation. Such an assay may be done as described above, e.g. using primary osteoclast progenitors.

A compound which exhibits this effect will further be tested in animal models which are useful to show that the compound has an *in vivo* inhibitory effect on bone resorption. Osteoporosis animal models are well known in the art, e.g. ovariectomized animals, or cytokines or hormone-treated animals.

Toxicity and therapeutic efficacy of the compounds identified as drug candidates by the method of the invention or by JNK1 antisense or ribozymes, can be determined by standard pharmaceutical procedures, which include conducting cell culture and animal experiments to determine the IC₅₀, LD₅0, the ED₅₀. The data obtained are used for determining the human dose range, which will also depend on the dosage form (tablets, capsules, aerosol sprays, ampules, etc.) and the administration route (oral, buccal, nasal, paterental or rectal). A pharmaceutical composition containing the compound as the active ingredient can be formulated in conventional manner using or more physologically active carriers and excipients. Methods for making such formulations can be found in manuals, e.g. "Remington Pharmaceutical Sciences". Examples for ingredients that are useful for formulating the compounds identified according to the present invention are also found in WO 99/18193.

### Brief description of the figures.

- Fig. 1:: TRANCE SPECIFICALLY ACTIVATES JNK-1 IN OSTEOCLAST PROGENITORS A) JNKs activation in response to TRANCE B) UV response in jnk1 -/- mice C) JNK-2 is expressed and functional in jnk1 -/- osteoclast progenitors
- Fig. 2:: JNK2 TARGETED DELETION DOES NOT AFFECT OSTEOCLAST DIFFERENTIATION OR ACTIVATION. A) Osteoclast differentiation monitored by TRAP staining B) Osteoclast activation monitored by bone resorption on bovine cortical bone slices
- Fig. 3:: DECREASED OSTEOCLAST DIFFERENTIATION IN jnk1 -/- MICE. A) Osteoclast differentiation monitored by TRAP staining B) Osteoclast activation monitored by bone resorption on bovine cortical bone slices
- Fig. 4:: c-jun AA MICE EXHIBIT REDUCED OSTEOCLASTOGENESIS. Osteoclast differentiation monitored by TRAP staining
- Fig. 5:: TRANCE INDUCES AP1 BINDING IN OSTEOCLAST PROGENITORS. A) Gel shift analysis for determining the kinetic of activation of AP1 binding activity compared to NFκB. B) Quantification of AP1 and NFκB binding activity using phosphoimager
- Fig. 6:: IDENTIFICATION OF THE JUN COMPONENTS OF THE TRANCE DEPENDENT AP1 BINDING IN OSTEOCLAST PROGENITORS

In the Examples, the following materials and methods were used:
a) Transgenic mice lacking c-Jun N-terminal phosphorylation were generated as described by Behrens et al., 1999.
b) Jnk1 -/- and jnk 2-/- mice were generated as described in Sabapathy et al., 1999.
c) Cell preparations
   For the coculture experiments, supportive primary osteoblasts were isolated by sequential digestion of new born calvaria and hematopoetic precursors were prepared as previously described. Osteoclast differentiation was induced by treatment of the coculture by a combination of 1, 25 dihydoxyvitamin D₃ (1, 25(OH)₂D₃, 10⁻⁸ M) and Dexamethasone (Dex 10⁻⁷ M). The cells were plated at the ratio 10 ⁶ hematopoetic cells for 10 ⁵ osteoblasts per well in 24 multiwells plates (Akatsu et al., 1992).
   The resorption activity of the *in vitro* generated osteoclasts was monitored on bovine cortical bone slices in the same culture conditions (Tanaka et al., 1996).
   For JNKs activation experiments, osteoclast progenitors were generated from a mixed cell population of bone marrow and spleen by pre-treatment of the culture with recombinant mouse macrophage colony stimulating factor (mCSF 20 ng/ml) (R & D System) for 2 days. After over night serum starvation in presence of mCSF, cells were stimulated by human recombinant TRANCE (Insight biotechnology LTD) as indicated.
   For AP1 binding activity, osteoclast progenitors were prepared in the same way, but TRANCE was added without serum starvation.
d) Nuclear extracts and gel electrophoresis mobility shift assay
   Nuclear extracts were prepared according to previously described procedures (Tugores et al., 1992). Binding reactions were carried out using 2.5 µg of nuclear extract proteins and 3x10⁴ c.p.m. of ³²P end-labelled double-stranded oligonucleotide with DNA polymerase I large (Klenow) (New England Biolab) fragment enzyme. Quantification was monitored after phophoimager (Molecular Dynamics) exposure. Jun components of AP1 binding activity were identified with specific antibodies directed against each member of the Jun family (SC44, SC43, SC73 and SC74 (Santa Cruz Biotechnology).
e) Immunoprecipitation-kinase assay
   JNKs activation was monitored in immunoprecipitation-kinase assay as previously described, using 100 µg of total extract with either JNK-1 specific antibody (Pharmingen) or a mixed of anti-JNK1, anti-JNK2 (Santa Cruz Biotechnology). Quantification was monitored after phophoimager exposure.

### Example 1

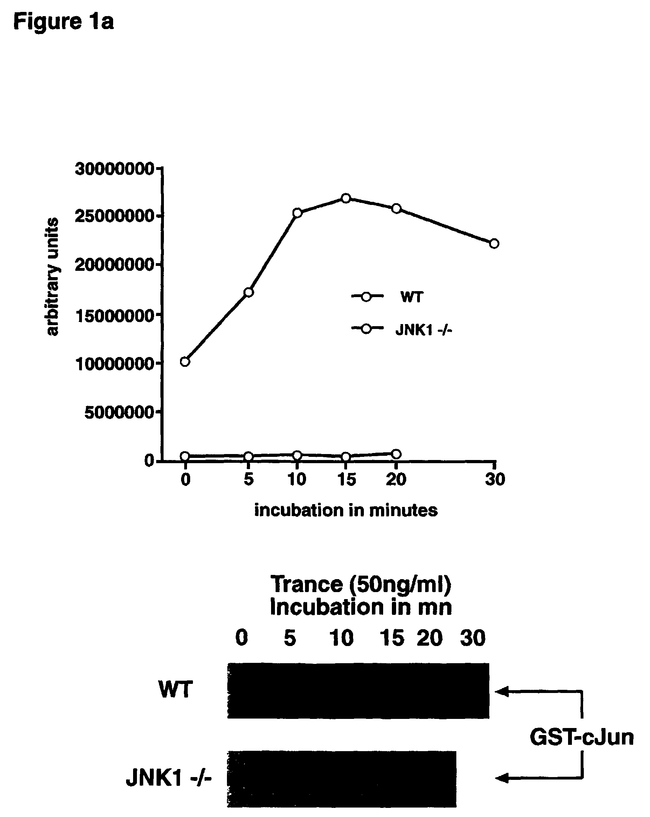

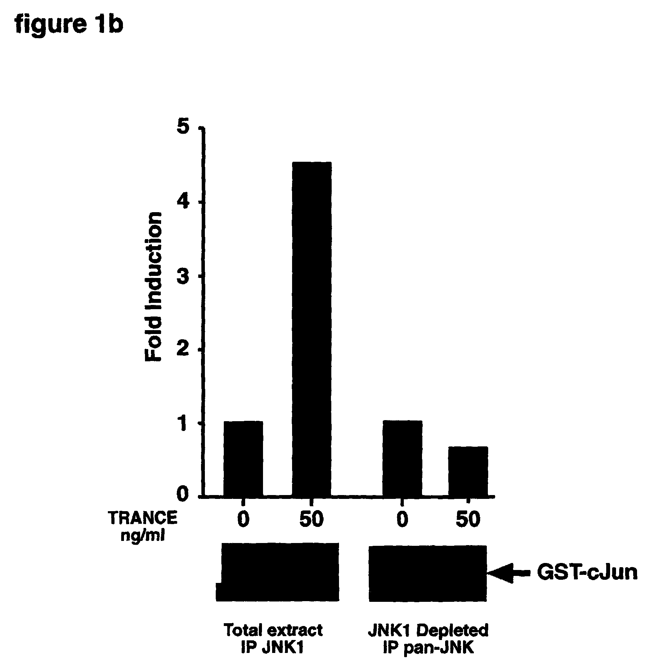

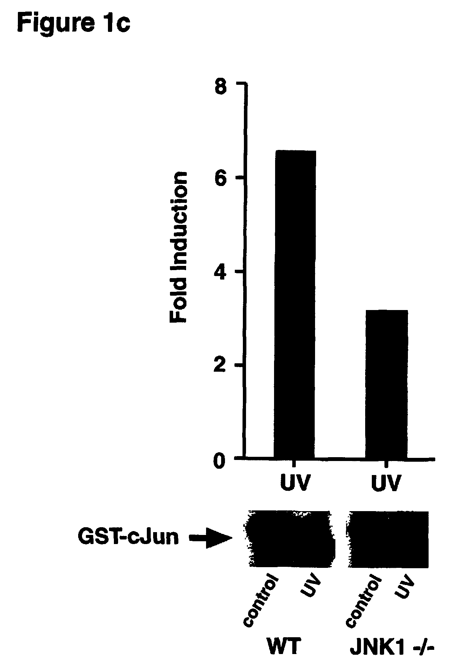

To determine whether TRANCE treatment leads to activation of JNKs during osteoclastogenesis, mCSF-dependent osteoclast progenitors were generated in vitro and, after serum starvation, treated with 50 ng/ml of soluble TRANCE for 20 minutes. JNKs activation were monitored in immunoprecipitation-kinase assay using GST-cJun as substrate. TRANCE treatment results in 2 to 5 fold activation of JNK (Fig. 1A and 1B). The JNKs activation is time dependent, and reach a maximal between 15 to 20 minute post TRANCE addition (Fig. 1A). JNK activity is immunoprecipitated by a specific antibody directed against JNK-1 isoforms (Fig. 1B) and not detected in the JNK-1 immunodepleted extract after immunoprcipitation with a mix of antibodies directed against JNK-1 and JNK-2 (IP pan JNK on Fig. 1B). Furthermore, JNKs activation in response to TRANCE is totally abolished in progenitors isolated from *jnk1* -/- mice (Fig. 1B) indicating that JNK-1 but not JNK-1 is activated in osteoclast progenitors. To determine whether the lack of activation of JNK-2 in response to TRANCE is due to the lack of expression of JNK-2 in these cells, osteoclast progenitors were treated with a potent activator of both isoforms (UV, 40 joules/cm²). UV stimulation results in a JNKs activation that could still, as expected, be detected in a lesser extend in osteoclast progenitors generated from *jnk1* -/- mice (Fig. 1C) indicating of the presence of functional JNK-2 that cannot compensate for the absence of JNK-1 in these cells. So TRANCE treatment of osteoclast progenitors results in a specific activation of JNK-1.

### Fig. 1:

A) Kinetic of JNKs activition in response to TRANCE: mCSF dependent osteoclast progenitors isolated from either wild type or *jnk1* -/- animals were incubated for various time as indicated with 50 ng/ml of purified recombinant TRANCE. JNKs activity was monitored after immunoprecipitation of total cellular exrtract with a mix of pan-JNK antibodies.
B) Specific JNK1 activation by TRANCE: Wild type mCSF dependent osteoclast progenitors were incubated for 20 minutes with 50 ng/ml of purified recombinant TRANCE. JNKs activation was measured either after immunoprecipitation with a specific antibody directed against JNK-1 (IP JNK1) or after JNK1 immunodepletion (IP pan-JNK).
C) JNK-2 is expressed and functional in *jnk1* -/- osteoclast progenitors: Osteoclast progenitors derived from *jnk1* -/- mice were prepared as described above and subjected to ultraviolet irradiation (40 Joules/cm²). After 30 minutes recovery, total cell extracts were prepared and JNKs activity mesured after immunoprecipitation using pan-JNKs antibodies mix. JNK activity was detected in *jnk1* -/- cells as expected at lower levels when compared to wild type cells, thus demonstrating the expression of a functional JNK2 in these cells.

### Example 2

Jnk2 deletion does not affect osteoclast differentiation and activation.

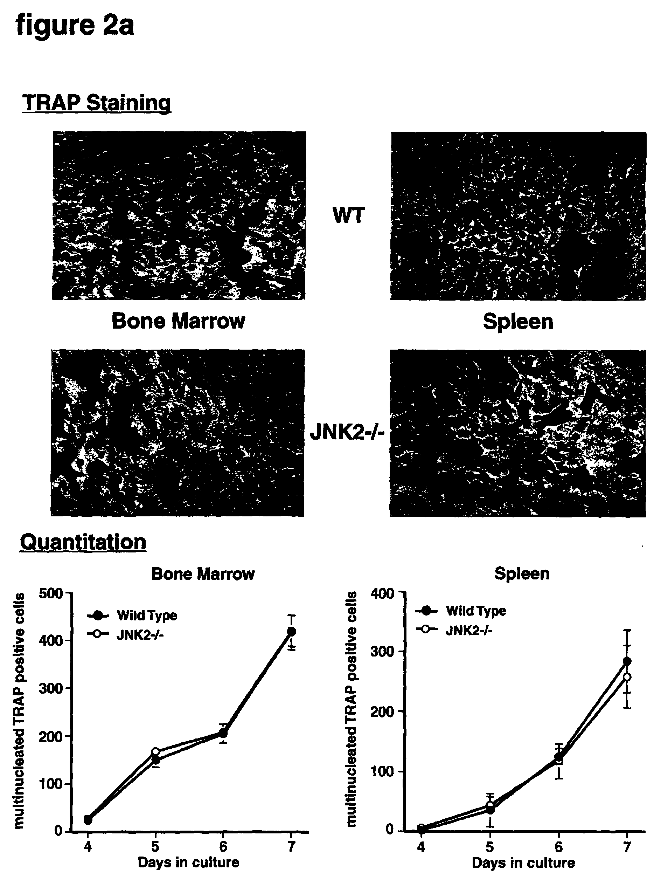

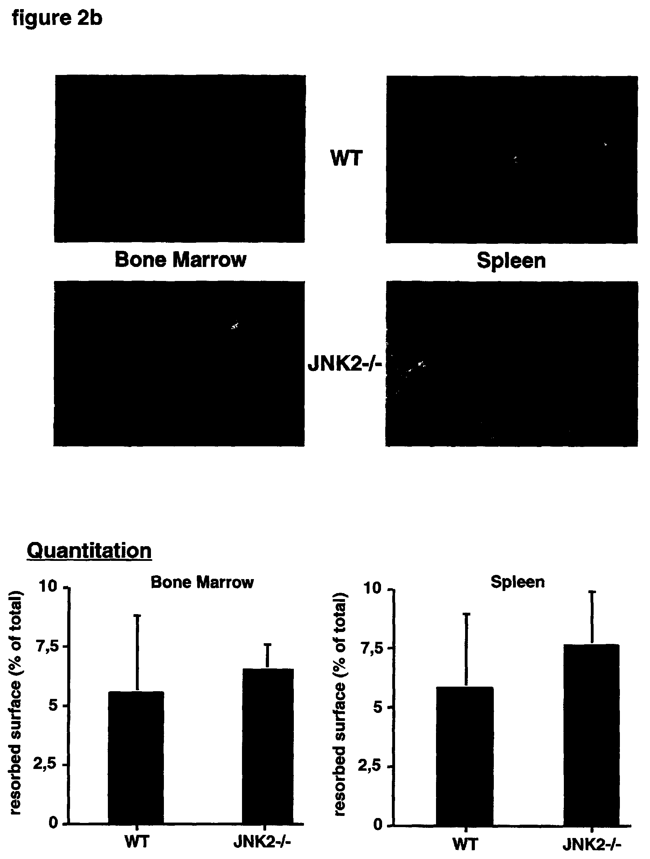

TRANCE treatment of osteoclast progenitors does not result in an activation of JNK-2 (Fig. 1) suggesting that JNK-2 does not play a role in osteoclast differentiation. The result is confirmed in coculture experiments where osteoclasts were generated by coculture of wild type or *jnk2* -/- hematopoetic precursors isolated from spleen or bone marrow on wild type osteoblasts. *jnk2* -/- hematopoetic precursors have the same differentiation properties as their wild type counterparts (Fig. 2A). Moreover, *jnk2* deletion does not affect the resorptive activity of the in vitro generated *jnk2* -/- osteoclasts (Fig. 2B), indicating that *jnk2* -/- osteoclasts are fully functional and that JNK2 has no role in osteoclast differentiation or activation.

### Fig. 2:

A) Osteoclast differentiation: Hematopoietic precursors derived from *jnk2* -/- mice were co-cultured for indicated time periods on supportive osteoblasts in conditions allowing osteoclast differentiation. Osteoclast differentiation was evaluated by TRAP staining and quantified by counting TRAP positive multinuclear cells (average of 5 randomly choosen fields). The results indicate that JNK 2 deletion does not affect osteoclasts differentiation.
B) Osteoclast activation: Activation of osteoclast was assessed by mesuring their bone resorbing capability on bovine cortical bone slices. For this purpose, the bone slices was added to the culture at day 1 until the end of the experiments. Bone resorption was evaluated by calculating the ratio of erroded bone surface rapported to the total surface. The results show that there is no difference between *jnk2*-/-osteoclasts and their wild type counterparts.

### Example 3

Decreased osteoclast differentiation in jnk1 -/- mice.

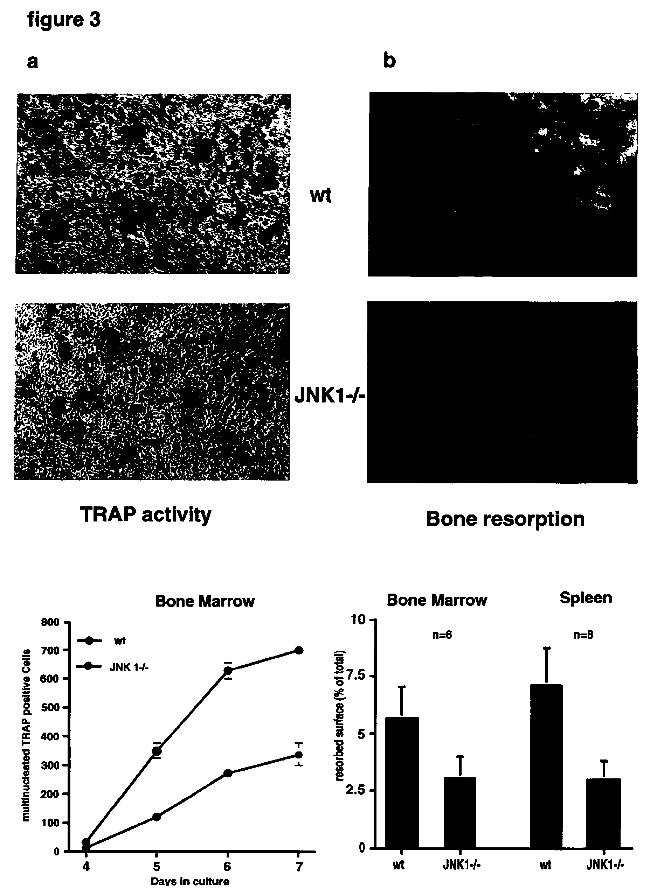

Identical experiments as in Example 2 were conducted to determine whether activation of JNK-1 in response to TRANCE could play a role in osteoclast differentiation and/or activation. Targeted deletion of *jnk1* results in a drastic reduction of the resorption activity due to a 50 % decreased in osteoclast differentiation efficiency when compared to wild type coculture (Fig. 3). The same results were obtained with hematopoetic cells isolated from spleen or bone marrow.

Fig 3: Osteoclast progenitors derived from *jnk1* -/- mice were prepared and treated as described in Example 2. The results demonstrate that the JNK1 null mutation decreases the differentiation capability of osteoclasts (50% of the wild type) (a) resulting in a concomitent decrease of the resorbing activity.

### Example 4

jun AA mice exhibit reduced osteoclastogenesis.

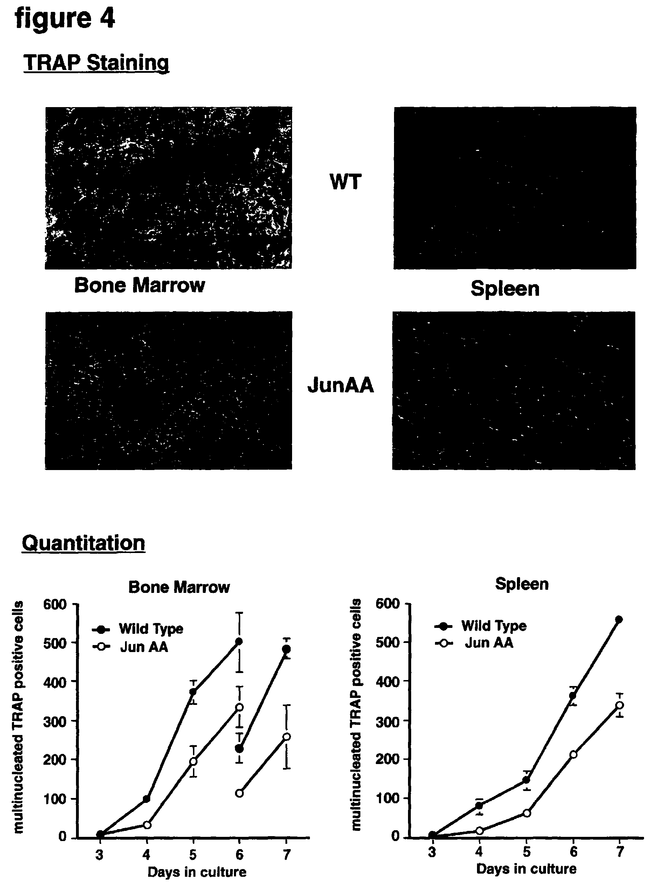

c-Jun is a potential substrate of the JNKs and was found to be expressed in osteoclasts. To determine whether c-Jun could be a potential substrate of JNK-1 in response to TRANCE during osteoclastogenesis, differentiation of hematopoetic precursors isolated from *jun* AA mice carrying double mutations of the 2 major JNKs phosphorylation sites was studied in coculture experiment. The differentiation ability of osteoclast progenitors derived from c-*jun* AA mice was evaluated as described in Example 2, the results show that c-*jun* AA osteoclast progenitors differentiation is impaired in a similar extent as *jnk1* -/- osteoclasts. As for jnk1 -/-, mutation of serine 63 and 73 results in a 50% decrease in osteoclasts differentiation (Fig. 4) indicating that c-Jun phosphorylation by JNK-1 in response to TRANCE is required for efficient osteoclastogenesis.

### Example 5

TRANCE induces AP1 binding activity in osteoclast progenitors

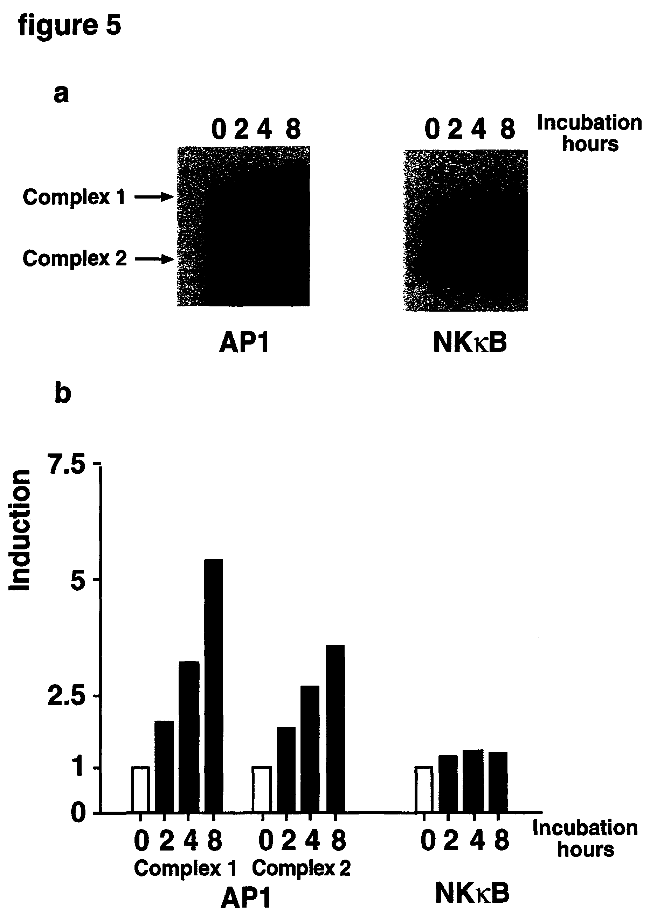

As TRANCE has originaly been characterized as a potent activator of NFκB, a key regulator of osteoclast differentiation (Kong et al., 1999), it was next determined whether TRANCE stimulation of osteoclast progenitors would lead to a modulation of NFκB and/or AP1 binding activity. TRANCE stimulation of mCSF dependent osteoclast progenitors results in a time dependent activation of AP1 binding, but dos not affect NFκB activation (Fig. 5). TRANCE also induces binding on cAMP responsive element (CRE), a target binding site for ATF/CREB family of transcription factors, another JNK dependent transcription factors complex .

### Fig. 5:

A) Kinetic of activation of AP1 binding activity compared to NFκB. mCSF dependent osteoclast progenitors were prepared as previously described and treated with TRANCE (50 ng/ml) for 0, 2, 4 and 8 hours before the preparation of nuclear extract. AP1 or NFκB binding activity was analyzed using 2.5 µl of nuclear extract. The results indicate that TRANCE stimulation of osteoclast progenitors induces a time dependent increase of AP1 binding activity, resolved as two different complexes, but does not affect NFκB binding activity.
B) Quantification of AP1 and NFκB binding activity using phosphoimager

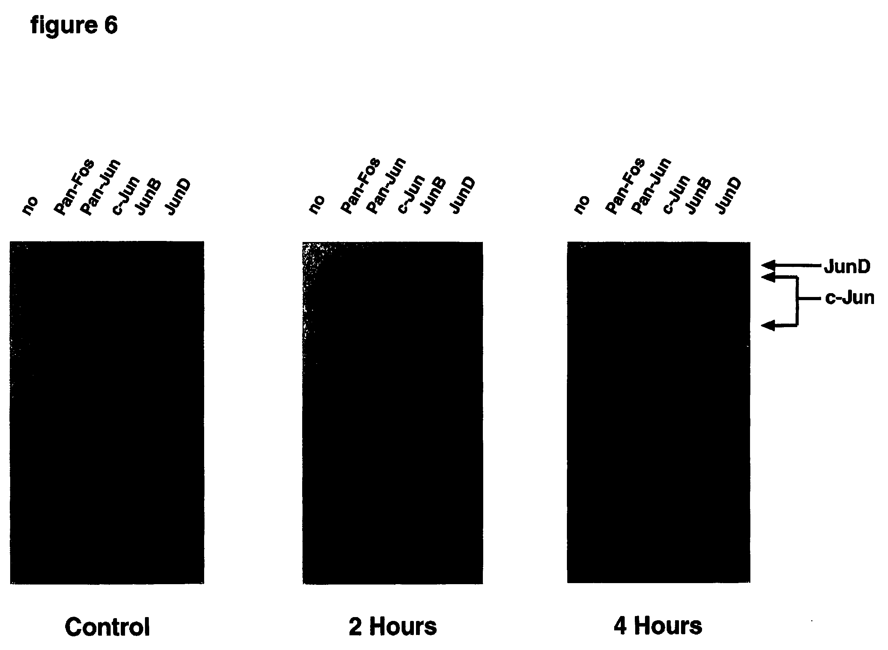

The Jun components of the TRANCE-induced AP1 binding activity were identified by super-shift as cJun and JunD, the two potential JNKs targets of the Jun family (Fig. 6).

Fig. 6: Jun components of AP1 binding activity induced by TRANCE in osteoclast progenitors were determined by super-shift experiments that demonstrated an increase in the amount of c-Jun and JunD bound to AP1 binding sites after 2 and 4 hours following TRANCE stimulation.

### References

Adler, V., et al., (1994). J Biol Chem, Vol. 269, 15, 11186-11191
Akatsu, T., Tamura, T., Takahashi, N., Udagawa, N., Tanaka, S., Sasaki, T., Yamaguchi, A., NAgata, N., and Suda, T. (1992). Preparation and characterization of a mouse osteoclast-like multinucleated cell population. J of Bone and Mineral Research 7, 1297-1306.
Anderson, D. M., Maraskovsky, E., Billingsley, W. L., Dougall, W. C., Tometsko, M. E., Roux, E. R., Teepe, M. C., DuBosse, R. F., Cosman, D., and Galibert, L. (1997). A homologue of the TNF receptor and its ligand enhance T-cell growth and dendritic-cell function. Nature *390*, 175-179.
Angel, P., and Karin, M. (1991). The role of Jun, Fos and the AP-1 complex in cell-proliferation an transformation. Biochemica et Biophysica Acta *1072*, 129-157.
Ausubel et al,, (1994). Current Protocols in Molecular Biology, John Wiley & Sons
Bauer, D., Muller, H., Reich, J., Riedel, H., Ahrenkiel, V., Warthoe, P., and Strauss, M., (1993). *Nucleic Acids Res*. 21, 4272-4280,
Behrens et al., (1999). Amino-terminal phosphorylation of c-Jun regulates stress-induced apoptosis and cellular proliferation. Nat Genet. Mar;21(3): 326-9.
Braselmann, S., Bergers, G., Wrighton, C., Graninger, Paula, Superti-Furga, G., and Busslinger, M. (1992). Identification of Fos target genes by the use of selective induction systems. Journal of Cell Science *16*, 97-109.
Cohen, D. R., and Curran, T. (1988). fra-1: a serum-inducible, cellular immediate-early gene that encodes a fos-related antigen. Molecular and Cellular Biology *8*, 2063-2069.
Darnay, B. G., Ni, J., Moore, P. A., and Aggarwal, B. B. (1999). Activation of NFκB by RANK requires tumor necrosis factor receptor-associated factor (TRAF) 6 and NFκB-inducing kinase. J of Biological Chemistry *274*, 7724-7731.
Davis, R. J. (1994). MAPKs: new JNK expands the group. TIBS *19*, 470-474.
Derijard et al., (1994). Cell, Mar 25;76(6):1025-37
DeRisi, J. L., et al., (1997). *Science*, 278, 680.
De Wet, J. R., et al., *Mol*. *Cell*. *Biol*. 7, 725-737(1987)
Diatchenko, L., Lau, Y.F., Campbell, A.P., Chenchik, A., Moquadam, F., Huang, B., Lukyanov, S., Lukyonov, K., Gurskaya, N., Sverdlov, E.D., and Siebert, P.D., (1996). *Proc*. *Natl*. *Acad*. *Sci*. *USA* 93, 6025-6030,.
Faris, M., *et al*., (1998). *J*. *Immunol*, 160, 134
Franzoso G, Carlson L, Xing L, Poljak L, Shores EW, Brown KD, Leonardi A, Tran T, Boyce BF, Siebenlist U. Requirement for NF-kappaB in osteoclast and B-cell development. Genes Dev 1997 Dec 15;11(24):3482-96
Galibert, l., Tometsko, M. E., Anderson, D. M., Cosman, D., and Dougall, W. C. (1998). The involvment of multiple tumor necrosis factor receptor (TNFR)-associated factors in the signaling mechanisms of the receptor activator of NF-kB, a member of the TNFR superfamily. J of Biological Chemistry *273*, 34120-34127.
Grigoriadis, A. E., Wang, Z.-Q., Cecchini, M. G., Hofstetter, W., Felix, R., Fleish, H. A., and Wagner, E. F. (1994). c-Fos: a key regulator of osteoclast-macrophage lineage determination and bone remodeling. Science *266*, 443-448.
Gupta, S., Barrett, T., Witmarsh, A. J., Cavanagh, J., Sluss, H. K., Derijard, B., and Davis, R. J. (1996). Slective interaction of JNK protein kinase isoforms with transcription factors. EMBO Journal *15*, 2760-2770.
Hattori, K., et al., PNAS, 85(23), 9148-9152 (1988)
Herdegen, T., *et al*., (1998). *J*. *Neurosci*., 18, 5124
Hubank, M. and Schatz, D.G., (1994), *Nucleic Acids Res*. 22, 5640-5648,
Inouye, S. and Tsuji, F. I., *FEBS Lett*. 341, 277-280 (1994)
Kallunki, T., et al., (1994). *Genes Dev*, Dec 15, 8(24), 2996-3007
Kasibhatla, S., *et al*., (1998). *Mol*. *Cell*, 1, 543
Kong, Y.-Y., Yoshida, H., Sarosi, I., Tan, H.-L., Timms, E., Capparelli, C., Morony, S., Oliveirados-Santos, A. J., Van, G., Itie, A., Khoo, W., Wakeham, A., Dunstan, C. R., Lacey, D. L., Mak, T. W., Boyle, W. J., and Penninger, J. M. (1999). OPGL is a key regulator of osteoclastogenesis, lymphocyte development and lymph-node organogenesis. Nature *397*, 315-323.
Kyriakis, J. M. *et al*., Nature, 369, 156 (1994)
Lacey, D. L., Timms, E., Tan, H.-L., Kelley, M. J., Dunstan, C. R., Burgess, T., Elliott, R., Colombero, A., Elliott, G., Scully, S., Hsu, H., Sullivan, J., Hawkins, N., Davy, E., Capparelli, C., Eli, A., qian, Y.-X., Kaufman, S., Sarosi, I., Shalhoub, V., Senaldi, G., Guo, J., Delaney, J., and Boyle, W. J. (1998). Osteoprotegerin ligand is a cytokine that regulates osteoclast differentiation and activation. Cell *93*, 165-176.
Liang, P. and Pardee, A.B., (1992). *Science* 257, 967-971.
Lomaga, M. A., Yeh, W.-C., Sarosi, I., Duncan, G., S., Furlonger, C., Ho, A., Morony, S., Capparelli, C., Van, G., Kaufman, S., van der Heiden, A., Itie, A., Wakehan, A., Khoo, W., Sasaki, T., Cao, Z., Penninger, J. M., Paige, C. J., Lacey, D. L., Dunstan, C. R., J., B. W., Goeddel, D. V., and Mak, T. W. (1999). TRAF6 deficiency results in osteoporosis and defective interleukin-1, CD40, and LPS signaling. Genes and Development *13*, 1015-1024.
Nagata, S., (1997). *Cell*, *88*, 355
Nakagawa N, Kinosaki M, Yamaguchi K, Shima N, Yasuda H, Yano K, Morinaga T, Higashio K., (1998). RANK is the essential signaling receptor for osteoclast differentiation factor in osteoclastogenesis. Biochem Biophys Res Commun Dec 18;253(2):395-400
Owen, J. M., Matsuo, K., Nicholson, G. C., Wagner, E. F., and Chambers, T. J. (1999). Fra-1 potentiates osteoclastic differentiation in osteoclastmacrophage precursor cell lines. Journal of cellular physiology *179*, 170-178.
Pasquali, C., et al., (1997). *Electrophoresis* 18, 2573-2581.
Pennington, S. R., et al., (1997), *Trends Cell Biol* 7, 168-173.
Ramsay, G., (1998). *Nature Biotechnol*., 16, 40
Sabapathy, K., Hu, Y., Kallunki, T., Schreiber, M., David, J.-P., Jochum, W., Wagner, E. F., and Karin, M. (1999). JNK2 is required for T-cell activation and apoptosis but not for normal lymphocyte development. Current Biology, Feb 11; 9(3):116-25
Sambrook, J., Fritsch, E.F. and Maniatis, T., (1989) Molecular Cloning, *Cold Spring Harbor Laboratory Press* (2. Edition),
Schena, M., et al., (1995). *Science* 270, 467
Sherr, C. J. (1991). Mitogenic response to colony-stimulating factor 1. Trends in Genetics *7*, 398-402.
Simonet, W. S., Lacey, D. L., Dunstan, C. R., Kelley, M., Chang, M.-S., Luthy, R., Nguyen, H. Q., Wooden, S., Bennett, L., Boone, T., Shimamoto, G., DeRose, M., Elliot, R., Colombero, A., Tan, H.-L., Trail, G., Sullivan, J., Davy, E., Bucay, N., Renshaw-Gegg, L., Hughes, T. M., Hill, D., Pattison, W., Campbell, P., Sander, S., Van, G., Tarpley, J., Derby, P., Lee, R., Program, A. E., and Boyle, W. J. (1997). Osteoprotegerin: a novel secreted protein involved in the regulation of bone density. Cell *89*, 309-319.
Soriano, P., Montgomery, C., Geske, R., and Bradley, A. (1991). Targeted disruption of the c-src proto-oncogene leads to osteoporosis in mice. Cell *64*, 693-702.
Takahashi, N., Udagawa, N., and Suda, T. (1999). A new member of tumor necrosis factor ligand family, ODF/OPGL/TRANCE/RANKL, regulates osteoclast differentiation and function. Biochemical and biophysical research communications *256*, 449-455.
Tanaka, S., Amling, M., Neff, L., Peyman, A., Uhlmann, E., Levy, J. B., and Baron, R. (1996). c-Cbl is dowtstreanm of c-Src in a signaling pathway necessary for bone resorption. nature *383*, 528-531.
Tugores, A., Alonso, M. A., Sanchez-Madrid, F., and de Landazuri, N. O. (1992). Human T cell activation through the activation-inducer AP-1. J of immunology *148*, 2300-2306.
Wang, Z.-Q., Ovitt, C., Grigoriadis, A. E., Mohle-Steinlein, U., Rither, U., and Wagner, E. F. (1992). Bone and hematopoietic defects in mice lacking c-fos. Nature *360*, 741-745.
Wodicka, L., et al., (1997). *Nature Biotechnol*., 15, 1359
Wong, B. R., Rho, J., Arron, J., Robinson, E., Orlinick, J., Chao, M., Lalachikov, S., Cayani, E., Barlett III, F. S., Frankel, W. N., Lee, S. Y., and Choi, Y. (1997). TRANCE is a novel ligand of the tumor necrosis factor receptor family that activates c-Jun N-terminal kinase in T cells. J of Biological Chemistry *272*, 25190-25194.
Yasuda, H., Shima, N., Nakagawa, N., Yamaguchi, K., Kinosaki, M., Mochizuki, S.-I., Tomoyasu, A., Yano, K., Goto, M., Murakami, A., Tsuda, E., Morinaga, T., Higashio, K., Udagawa, N., Takahashi, N., and Suda, T. (1998). Osteoclast differentiation factor is a ligand for osteoprotegerin/osteoclastogenesis-inhibitory factor and is identical to TRANCE/RANKL. PNAS *95*, 3597-3602.

## Claims

1. A method for identifying a compound useful for the treatment of bone disorders caused by osteoclast differentiation or activation that leads to increased bone resorption, wherein the compund is tested for its ability to inhibit JNK1 synthesis or JNK1 activation and/or c-Jun N-terminal phosphorylation.

2. The method of claim 1, wherein, upon incubation with the test compound, the inhibition of the physical interaction of JNK1 with one of its substrates to which it binds is detected.

3. The method of claim 2, wherein the substrate is c-Jun.

4. The method of claim 2 or 3, wherein the JNK1 binding domain of the substrate and the substrate binding domain on JNK1, one of them being fused to a detectable reporter molecule, and the other one being immobilized on a suitable carrier, are incubated with the test compound and the effect of the compound on the interaction of the two proteins is determined by measuring the reporter activity.

5. The method of claim 1, wherein the method comprises a cell-free kinase assay in which the modulating effect of the test compound on the phosphorylation of a JNK1 substrate by JNK1 is determined.

6. The method of claim 5, wherein the modulating effect of the test compound on the N-terminal of c-Jun by JNK1 is determined.

7. The method of claim 5, wherein recombinant JNK1 and a recombinant JNK1 substrate are incubated with the test compound in the presence of adenosine triphosphate (ATP), the substrate being immobilized on a suitable carrier, and the effect of the compound on the kinase reaction is determined by measuring the incorporation of phosphate into the substrate.

8. The method of claim 6, wherein the substrate is c-Jun.

9. The method of claim 1, wherein a compound is identified by its ability to transcriptionally downregulate *jnk1* expression.

10. The method of claim 8, said method comprising incubating mammalian cells that are transfected with a plasmid carrying a reporter gene under the control of regulatory elements from the *jnk1* gene with a number of test compounds and selecting a compound which downregulates the expression of the reporter gene.

11. The method of claim 1, wherein c-Jun N-terminal phosphorylation or JNK1-dependent substrate activation is monitored by determining the expression of a reporter gene under the control of a regulatory element of a c-Jun target gene, which is directly regulated by this mechanism.

12. The method of claim 10, wherein the c-Jun target gene is fasL or collagenase I.

13. The method of claim 11, wherein the reporter gene is under the control of a target gene of a JNK1 substrate different from c-jun.

14. The method of claim 1, wherein osteoclast progenitors are incubated with a compound and and the compound is selected due to its ability to decrease osteoclast differentiation and activation.

15. The method of claim 13, wherein osteoclast progenitors are cocultured and induced to differentiate on osteoblastic supportive cells, incubated with the test compound and the compound is selected due to its ability to decrease osteoclast differentiation or activation.

16. The method of claim 14 or 15, wherein the osteoclast progenitors are hematopoietic cells from bone marrow or spleen cells.

17. The method of claim 14, 15 or 16, wherein the supportive cells are osteoblasts.

18. The method of claim 13, wherein mCSF dependent osteoclast precursors stimulated with TRANCE are incubated with the compound.

19. A JNK1 inhibitor for the treatment of bone disorders caused by osteoclast differentiation or activation that leads to increased bone resorption.

20. The JNK1 inhibitor of claim 19 which is a JNK1 antisense oligonucleotide molecule.

21. An inhibitor of c-Jun N-terminal phosphorylation for the treatment of bone disorders caused by osteoclast differentiation or activation that leads to increased bone resorption.

22. An inhibitor of claim 19 or 21 for the treatment of osteoporosis.
